(19) Europäisches Patentamt
European Patent Office
Office européen des brevets

(11) Publication number: **0 185 401**
**B1**

(12) # EUROPEAN PATENT SPECIFICATION

(45) Date of publication of patent specification: **06.06.90**

(51) Int. Cl.$^5$: **A 01 N 43/647,**
A 01 N 43/653,
C 07 D 249/12,
C 07 D 249/14,
C 07 D 249/06,
C 07 D 249/08, C 07 D 401/12

(21) Application number: **85201763.1**

(22) Date of filing: **30.10.85**

(54) **Heterocyclic herbicides.**

(30) Priority: **20.11.84 GB 8429307**

(43) Date of publication of application:
**25.06.86 Bulletin 86/26**

(45) Publication of the grant of the patent:
**06.06.90 Bulletin 90/23**

(84) Designated Contracting States:
**AT BE CH DE FR GB IT LI LU NL SE**

(56) References cited:
**DE-A- 818 048**
**FR-A-2 268 018**
**GB-A-1 288 540**
**GB-A-2 070 607**
**US-A-3 470 196**
**US-A-3 489 761**

**CHEMICAL ABSTRACTS, vol. 57, no. 10, 12th November 1962, no. 12473b, Columbus, Ohio, US; S. NAQUI et al.: "Replacement of the hydroxyl function in 3,4-diaryl-5-hydroxy-1,2,4-triazoles", & J. SCI. IND. RES. (India) 21B, no. 4, 195-6, 1962**

**INDUSTRIE CHIMIQUE BELGE, vol. 36, no. 1, January 1971, pages 3-10, Brussel, BE; G. L'ABBE: "The dimroth reaction"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Mansuri, Muzammil Mansoor**
**207-208 Highbridge Street**
**Fayetteville New York 13066 (US)**
Inventor: **McArthur, Alasdair**
**31 Heathedge Kirkdale**
**Sydenham London SE26 (GB)**

(74) Representative: **Bennett, David Arthur Horder et al**
**4, York Road**
**London SE1 7NA (GB)**

(56) References cited:
**CHEMICAL ABSTRACTS, vol. 95, no. 13, 28th September 1981, page 710, no. 115565s, Columbus, Ohio, US; & JP - A - 81 49 371 (NIHON NOHYAKU CO., LTD.) 02-05-1981**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 185 401 B1

**Description**

This invention relates to a composition for and a method of controlling undesired plant growth and to compounds for use in such a composition or method.

US Patent Specification No. 3489761 describes certain substituted N-phenyl 1,2,4-triazoles which are shown primarily to be useful as antifertility agents for the control of animal populations and which are stated to have biological activity when at least one nuclear carbon atom is unsubstituted.

3-phenoxy-2,5-diphenyl and 3-phenoxy-4,5-diphenyl-1,2,4-triazoles are disclosed in Chem. Abs. 57, 12473c, and 64, 8171e. Also, Japanese Patent Application Publication No. 56049371 of Nihon Noyaku discloses certain N-phenyl 1,2,4-triazoles having a very specific substitution pattern on the phenyl ring, including N-(2,4-dichloro-5-methoxyphenyl)-1,2,4-triazoles, and describes their use in controlling plant fungal diseases. There is no teaching in these references that N-phenyl-C-phenoxy triazoles have any herbicidal activity; in fact the disclosure of plant protectant applications in the Japanese patent leads directly away from the idea of herbicidal activity.

British Patent Specification No. 1288540 describes certain N-substituted 1,3,4-triazoles having an optional thio substituent which are useful as fungicides and a more restricted group of 1,3,4-triazoles substituted only at the 1-nitrogen atom which are useful for combating undesired vegetation. The disclosures of the British Specification lead away from the idea that triazoles substituted other than at the 1-nitrogen atom may have herbicidal activity.

It has now surprisingly been found that certain substituted N-aryl triazoles do indeed have useful herbicidal properties, and accordingly the present invention provides a herbicidal composition which comprises a carrier and, as active ingredient, a N-aryl triazole of the formula I:—

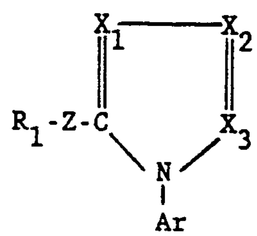

I

wherein $X_3$ and one of $X_1$ and $X_2$ represent a nitrogen atom and the other of $X_1$ and $X_2$ represents a group C—$R_2$;

Z represents an imino group or a sulphur or oxygen atom;

$R_1$ represents an optionally substituted alkyl, aralkyl, alkenyl, cycloalkyl, cycloalkenyl, heteroaryl, preferably pyridyl, or aryl, preferably phenyl group;

$R_2$ represents a hydrogen or halogen atom or an optionally substituted aryl or alkyl group;

and Ar represents an optionally substituted aryl, preferably phenyl, group.

When any of the foregoing substituents represents or contains an alkyl or alkenyl substituent group, this may be linear or branched and may contain up to 12, preferably up to 6, and especially from 1 to 4, carbon atoms, suitable examples being methyl, ethyl and propyl. When they represent or contain a cycloalkyl or cycloalkenyl substituent group this may contain from 3 to 10, especially 5 to 8, carbon atoms, and is preferably cyclohexyl, methylcyclohexyl or cyclohexenyl. When they contain an aryl substituent group, this may be polynuclear such as naphthalene, but is preferably a phenyl group. When they contain a heteroaryl group, this may, for example, be a pyrrole, quinoline, furan, pyran, or, preferably, a pyridine ring. When any of the foregoing substituents are designated as being optionally substituted, the substituent groups which are optionally present may be any of these customarily employed in the development of pesticidal compounds, and/or the modification of such compounds to influence their structure/activity,. persistence, penetration or other property. Specific examples of such substituents include halogen, especially fluorine, chlorine or bromine, atoms, and nitro, cyano, hydroxyl, alkyl, haloalkyl, alkoxy and alkoxycarbonyl groups; in the case of halogen substituted alkyl groups, a particular preferred examples is trifluoromethyl.

It will be appreciated that the alternative meanings for $X_1$, $X_2$ and $X_3$ correspond to the different isomeric forms of the N-substituted triazole riong; of the 2 isomeric configurations the 1, 2, 4 configuration is preferred.

Preferably Z represents an oxygen atom; $R_1$ represents a $C_{1-4}$ alkyl or haloalkyl, especially methyl, propyl, fluoropropyl or fluorobutyl group, a cycloalkyl or cycloalkenyl group of 5 to 8 carbon atoms, especially cyclohexyl, methylcyclohexyl, or cyclohexenyl, a pyridyl group, a naphthyl group, or a phenyl group optionally substituted by a fluorine, chlorine or bromine atom or a hydroxy, cyano, methyl, trifluoromethyl, methoxy, or methoxycarbonyl group; $R_2$ represents a hydrogen or halogen, especially chlorine, atom, a $C_{1-4}$ alkyl, especially methyl, group; or a phenyl group; and Ar represents a phenyl group optionally substituted by a halogen, especially chlorine, atom or an alkyl or haloalkyl group of 1 to 4 carbon atoms, especially methyl or trifluoromethyl, which is desirably located at the 3-position.

Many of the compounds of formula I are novel and the invention therefore also extends to these novel compounds *per se.* The novel compounds are the N-aryl triazoles of formula I wherein the substituents $X_1$, $X_2$, $X_3$, Ar, $R_1$ and Z have the meanings defined above, with the provisos that when $X_2$ represents C-Phenyl and $R_2$Z represents phenoxy, then Ar is not unsubstituted phenyl; and when $X_2$ represents CH, then Ar is not a 2,4-dichloro-5-methoxy-phenyl group.

The invention also provides a process for the preparation of compounds of the general formula I in which Z represents an oxygen or sulphur atom, which comprises reacting a halo-triazole of formula II:—

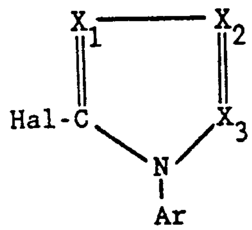

II

with an alcohol or mercaptan of formula $R_1ZH$, or an alkali metal salt of such an alcohol or mercaptan, wherein $X_1$, $X_2$, $X_3$, Ar, $R_1$ and Z are as defined above and Hal denotes a halogen, preferably chlorine or bromine, atom. This reaction is suitably carried out in an organic solvent, in particular a polar solvent such dimethyl formamide, and preferably in the presence of a base, such as an alkali metal alkoxide, e.g. potassium tert. butoxide, or potassium carbonate.

The halotriazole may be prepared by appropriate adaptations of established synthetic methods, which will often differ for the different isomeric forms of the triazole ring. Thus, the 1-aryl and 4-aryl 1,2,4 halotriazoles may be prepared by reacting a semicarbazide of formula $H_2N—CO—N(Ar)—NH_2$ or $ArNHCONHNH_2$, respectively, with an alkyl orthoformate of formula $R_2C(OAlkyl)_3$, followed by halogenation, suitably with a phosphorus oxyhalide such as phosphorus oxychloride. An alternative route to the 1,2,4 halotriazoles is by dissolving cyanauric chloride in dimethylformamide followed by reaction with an arylhydrazine of formula $ArNHNH_2$ and N-bromosuccinimide.

In the case of the 1,2,3 triazoles of formula I above, a suitable synthetic process is direct cyclization of an azide of formula $ArN_3$, which can suitably be effected by reaction with an acetylenic derivative of formula $R_1—Z—C{=}CH$, or by reaction with a dialkyl malonate, followed by treatment with a phosphorus pentahalide, an alcohol or mercaptan $R_1ZH$ and removal of the ester group by hydrolysis and heating.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilizers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilizers, surfactants, slow release modifiers and binding agents. The so-called "dry flowable powders" consist of relatively small granules having a relatively high concentration of active ingredient. Emulsifiable concentrates usually contain, in addition to solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following Examples.

Example 1

Preparation of 1-Phenyl-3-methyl-5-phenoxy-1,2,4-triazole via semicarbazide route

A) 2-Phenylsemicarbazide (6.95 g) was reacted with triethyl orthoacetate (7.45 g) in 2-methoxyethanol (20 ml) under reflux with stirring, and ethanol distilled off. The reaction mixture was concentrated, and the product chromatographically purified to yield, as a solid m.pt. 163—165°C the triazolinone of structure:

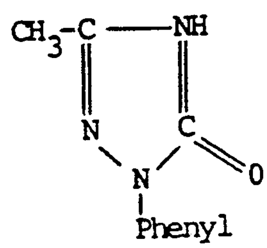

B) The triazolinone of A) (3.0 g) was heated with phosphoryl chloride (5 ml) in a sealed tube at 200°C for 2 hours. After cooling, the reaction mixture was poured into ice water, the crude product collected by filtration and purified chromatography to yield 1-phenyl-3-methyl-5-chloro-1,2,4 triazole as a solid, m.pt. 81—84°C.

C) Potassium tert. butoxide (0.93 g) was added to a stirred solution of phenol (0.71 g) in dry dimethyl formamide (15 ml). When all the butoxide had dissolved, a solution of the chloro-triazole produced in B) (1.45 g) in dry dimethylformamide (10 ml) was added and the mixture stirred and heated at 80°C for 2 hours. After cooling, the reaction mixture was poured into water and extracted with ether. The ether extracts were washed with 5% aqueous sodium hydroxide, dried and evaporated to yield the desired product, which was recrystallised from petroleum ether (40°—60°) to form a solid, m.pt 64—66°C.

Analysis

| | | | |
|---|---|---|---|
| Calculated: | C 71.7; | H 5.2; | N 16.7 |
| Found: | C 71.7; | H 5.1; | N 16.7 |

Example 2

Preparation of 1-(3-trifluoromethylphenyl)-5-phenoxy-1,2,4-triazole via cyanuric chloride route

A) Cyanuric chloride (36.8 g) was dissolved in dimethylformamide (120 ml) and the solution stirred at room temperature. After about 10 minutes an exothermic reaction started and a precipitate formed. The temperature was maintained at 50—60°C, when the precipitate gradually dissolved and carbon dioxide evolved. When evolution of carbon dioxide had ceased, the mixture was cooled and the crystalline product triturated with acetone, filtered, washed with acetone and dried.

B) The dimethylammonium salt of A) (16.4 g) and 3-trifluoromethylphenylhydrazine were stirred and heated to 60°C. Dimethylamine was evolved in an exothermic reaction and the temperature maintained at 90°C until evolution ceased (about 30 minutes). After cooling, the reaction mixture was dissolved in ether, washed with water, dried and chromatographically purified to yield 1-(3-trifluoromethylphenyl)-1,2,4 triazole.

C) the product of B) (8.52 g) was mixed with N-bromosuccinimide (7.83 g) and benzoyl peroxide (c. 50 mg) in carbon tetrachloride (250 ml). The mixture was stirred under reflux for 3 hours. After cooling, the reaction mixture was filtered and the filtrate evaporated to yield the bromotriazole as a brown oil, which was used directly for the next stage.

D) Potassium tert. butoxide (1.0 g) was added to a solution of phenol (0.75 g) in dry dimethylformamide under a nitrogen atmosphere, and when the base had dissolved a solution of the bromotriazole product of C) (2.3 g) in dry dimethylformamide (10 ml) was added. The reaction mixture was stirred at 70—80°C for 2 hours, cooled and poured into ice/water. After standing, the solid product was filtered from the aqueous mixture, washed and dried to yield the desired product, m.pt. 43—45°C.

The same product was also prepared by the procedure of Example 1, using 2-(3-trifluoromethylphenyl)semicarbazide and triethyl orthoformate as starting materials. This product had a m.pt. 46—48°C.

Analysis

| | | | |
|---|---|---|---|
| Calculated: | C 59.0; | H 3.3; | N 13.8 |
| Found: | C 58.9; | H 3.2; | N 13.7 |

Examples 3 to 37

Following procedures analogous to those described in Examples 1 and 2, further 1,2,4 triazole derivatives were prepared, whose physical characteristics and analyses are given in Table 1 below. In this Table, the compounds are identified by reference to the substituents in the formula

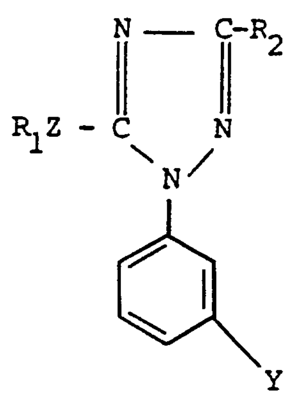

TABLE 1

| Ex. No. | $R_1$ | Z | Y | $R_2$ | M.Pt. | Analysis | | | | | |
|---|---|---|---|---|---|---|---|---|---|---|---|
| | | | | | | Calc. | | | Found | | |
| | | | | | | C | H | N | C | H | N |
| 3 | Phenyl | O | H | H | 48-50 | 70.9 | 4.6 | 17.7 | 70.0 | 4.7 | 17.7 |
| 4 | Phenyl | O | $CF_3$ | $CH_3$ | 73-75 | 60.2 | 3.8 | 13.2 | 60.1 | 3.6 | 12.9 |
| 5 | m-F-Phenyl | O | $CF_3$ | H | 53-54 | 55.7 | 2.8 | 13.0 | 55.8 | 3.0 | 13.0 |
| 6 | p-F-Phenyl | O | $CF_3$ | H | 59-60 | 55.7 | 2.8 | 13.0 | 55.4 | 2.5 | 12.8 |
| 7 | o-F-Phenyl | O | $CF_3$ | H | 41-43 | 55.7 | 2.8 | 13.0 | 55.7 | 3.0 | 13.0 |
| 8 | m-Cl-Phenyl | O | $CF_3$ | H | 70-72 | 53.0 | 2.7 | 12.4 | 53.1 | 2.5 | 12.2 |
| 9 | m-$CH_3$-Phenyl | O | $CF_3$ | H | 48-52 | 60.2 | 3.8 | 13.2 | 59.7 | 3.6 | 13.0 |
| 10 | p-$CH_3$-Phenyl | O | $CF_3$ | H | 65-67 | 60.2 | 3.8 | 13.2 | 58.6 | 4.1 | 12.8 |
| 11 | $CH_3$ | O | $CF_3$ | H | 60-61 | 49.4 | 3.3 | 17.3 | 49.1 | 3.3 | 17.0 |
| 12 | Cyclohexyl | O | $CF_3$ | H | oil | | | | | | |
| 13 | iso-$C_3H_7$ | O | $CF_3$ | H | oil | 53.1 | 4.5 | 15.5 | 53.2 | 4.6 | 15.3 |
| 14 | o-$CH_3$-Phenyl | O | $CF_3$ | H | 55-58 | 60.2 | 3.8 | 13.2 | 60.0 | 3.7 | 12.8 |
| 15 | 3,5-$Cl_2$-Phenyl | O | $CF_3$ | H | 88-89 | 48.1 | 2.2 | 11.2 | 48.4 | 1.9 | 10.8 |
| 16 | m-$CF_3$-Phenyl | O | $CF_3$ | H | 58-59 | 51.5 | 2.4 | 11.3 | 51.3 | 2.4 | 11.0 |

Table 1 (cont'd)

| Ex. No. | $R_1$ | Z | Y | $R_2$ | M.Pt. | Analysis | | | | | |
|---|---|---|---|---|---|---|---|---|---|---|---|
| | | | | | | Calc. | | | Found | | |
| | | | | | | C | H | N | C | H | N |
| 17 | m-CN-Phenyl | O | $CF_3$ | H | 105-106 | 58.2 | 2.7 | 16.9 | 58.1 | 2.6 | 16.9 |
| 18 | m-Br-Phenyl | O | $CF_3$ | H | 70-72 | 46.9 | 2.4 | 10.9 | 47.1 | 2.2 | 10.8 |
| 19 | m-$CH_3$O-Phenyl | O | $CF_3$ | H | 45-47 | 57.3 | 3.6 | 12.5 | 57.4 | 3.3 | 12.6 |
| 20 | 3-Pyridyl | O | $CF_3$ | H | 52-53 | 54.9 | 2.9 | 18.3 | 54.7 | 2.9 | 18.1 |
| 21 | 2-Naphthyl | O | $CF_3$ | H | 93-94 | 64.2 | 3.4 | 11.8 | 63.9 | 3.5 | 11.8 |
| 22 | Phenyl | S | $CF_3$ | H | 84-85 | 56.1 | 3.1 | 13.1 | 56.3 | 3.1 | 13.1 |
| 23 | Phenyl | NH | $CF_3$ | H | 67-72 | 59.2 | 3.6 | 18.4 | 59.9 | 4.1 | 17.7 |
| 24 | Phenyl (HCl) | NH | $CF_3$ | H | | 52.3 | 3.5 | 16.4 | 55.8 | 3.7 | 17.4 |
| 25 | $C_3H_7n$ | NH | $CF_3$ | H | oil | 53.3 | 4.8 | 20.7 | 53.1 | 5.5 | 19.0 |
| 26 | Phenyl | O | $CF_3$ | Phenyl | 93-94 | 66.1 | 3.7 | 11.0 | 66.1 | 3.6 | 11.0 |
| 27 | m-$COOCH_3$Ph | O | $CF_3$ | H | 46-47 | 56.2 | 3.3 | 11.6 | 56.1 | 3.4 | 11.4 |
| 28 | 3-$CH_3$cyclohexyl (cis) | O | $CF_3$ | H | oil | 59.0 | 5.6 | 12.9 | 59.1 | 5.7 | 13.0 |

Table 1 (cont'd)

| Ex. No. | $R_1$ | Z | Y | $R_2$ | M.Pt. | Analysis Calc. C | Calc. H | Calc. N | Found C | Found H | Found N |
|---|---|---|---|---|---|---|---|---|---|---|---|
| 29 | 3-$CH_3$cyclohexyl (trans) | O | $CF_3$ | H | oil | 59.0 | 5.6 | 12.9 | 59.1 | 5.5 | 13.0 |
| 30 | 2-$CH_3$cyclohexyl (trans) | O | $CF_3$ | H | oil | 59.0 | 5.6 | 12.9 | 59.1 | 5.7 | 12.8 |
| 31 | 2-$CH_3$cyclohexyl (cis) | O | $CF_3$ | H | oil | 59.0 | 5.6 | 12.9 | 57.2 | 6.0 | 10.8 |
| 32 | 3,5-di$CH_3$cyclohexyl | O | $CF_3$ | H | oil | 60.2 | 5.9 | 12.4 | 57.7 | 6.1 | 11.3 |
| 33 | 2-cyclohexenyl | O | $CF_3$ | H | 82-83 | 58.2 | 4.5 | 13.6 | 58.2 | 4.7 | 13.4 |
| 34 | $(CF_3)_2CH$ | O | $CF_3$ | H | 41-43 | 38.0 | 1.6 | 11.1 | 38.5 | 1.6 | 10.2 |
| 35 | $C_3F_7CH_2$ | O | $CF_3$ | H | oil | 38.0 | 1.7 | 10.2 | 38.1 | 2.1 | 9.0 |
| 36 | Phenyl | O | $CF_3$ | Cl | 65-66 | 61.9 | 3.7 | 15.5 | 61.8 | 3.7 | 15.8 |
| 37 | Phenyl | O | $CF_3$ | Cl | 85-87 | 53.0 | 2.7 | 12.4 | 53.0 | 2.8 | 12.4 |

Example 38

Preparation of 1-Phenyl-5-phenoxy-1,2,3-triazole form phenoxyacetylene

A mixture of phenylazide (2.6 g) and phenoxyacetylene (2.36 g) in benzene (20 ml) was stirred under reflux overnight. The reaction mixture was chromatographically separated to yield the desired product as a solid, m.pt. 96—98°C.

Analysis

| | | | |
|---|---|---|---|
| Calculated: | C 70.9; | H 4.6; | N 17.7 |
| Found: | C 71.0; | H 4.5; | N 17.6 |

Example 39

Following a similar procedure to Example 26, but using 3-trifluoromethylphenylazide, 1-(3-trifluoromethylphenyl)-5-phenoxy-1,2,3-triazole was obtained as an oil.

Analysis

| | | | |
|---|---|---|---|
| Calculated: | C 59.0% | H 3.3%; | N 13.8% |
| Found: | C 59.5% | H 3.6; | N 13.6% |

Example 40

Preparation of 1-Phenyl-5-phenoxy-1,2,3-triazole from diethyl malonate and phenyl azide

A) Diethyl malonate (32 g) was added to a stirred solution of sodium ethoxide (4.6 g sodium in 80 ml ethanol). Phenylazide (23.8 g) was then added dropwise with cooling, after which the reaction mixture was refluxed for 30 minutes. After cooling, the sodium salt of the enol ester formed was filtered off, dissolved in water, washed with ether, and acidified with dilute hydrochloric acid. The resultant yellow oil was recrystallised to yield 1-phenyl-4-ethoxy-carbonyl-5-hydroxy 1,2,3-triazole, m.pt. 72—74°C.

B) The hydroxy ester product of A) (11.65 g) was mixed with phosphorus pentachloride, and slowly heated to c. 70°C, when vigorous reaction occurred. When reaction had subsided, heating was continued until evolution of hydrogen chloride ceased (about 1 hour), after which phosphoryl chloride was removed *in vacuo*, and the residue chromatographically purified to yield 1-phenyl-4-ethoxycarbonyl-5-chloro-1,2,3-triazole, m.pt. 80—81°C.

C) Potassium tert. butoxide (2.5 g) was added to a stirred solution of phenol (1.9 g) in dry dimethylformamide (45 ml). The chloroester product of B) (5.0 g) in dry dimethylformamide (40 ml) was added to the resulting phenoxide solution and the mixture stirred and heated at 80°C for 2 hours. After cooling, the reaction mixture was poured into water, and the solid product filtered off, washed with water, and chromatographically purified to yield the 5-phenoxy derivative, m.pt 91—94°C.

D) The ester of C) (4 g) was hydrolysed with excess aqueous 1.25M sodium hydroxide, and the product acidified with dilute hydrochloric acid to yield 1-phenyl-4-carboxyl-5-phenoxy-1,2,3-triazole.

E) The phenoxy acid of D) was heated until evolution of carbon dioxide ceased, and the product purified chromatographically to yield the title product, m.pt. 96—98°C.

Analysis

| | | | |
|---|---|---|---|
| Calculated: | C 70.9; | H 4.6; | N 17.7 |
| Found: | C 70.9; | H 4.5; | N 17.5 |

Example 41—46

Following procedures similar to those described in Examples 38—40, further 1, 2, 3 triazole derivatives were prepared, whose physical characteristics and analyses are given in Table 2 below. In this Table the compounds are identified by reference to the substituents in the formula

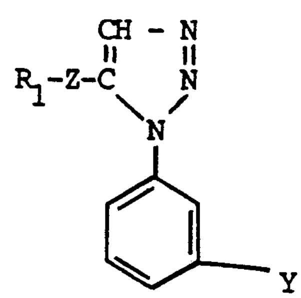

TABLE 2

| Ex. No. | $R_1$ | Z | Y | $R_2$ | M.Pt. °C | Analysis | | | | | |
|---|---|---|---|---|---|---|---|---|---|---|---|
| | | | | | | Calc. | | | Found | | |
| | | | | | | C | H | N | C | H | N |
| 41 | 2-OH-Phenyl | O | $CF_3$ | H | 147-148 | 56.1 | 3.1 | 13.1 | 56.0 | 3.2 | 13.2 |
| 42 | 3-F-Phenyl | O | $CF_3$ | H | oil | 55.7 | 2.8 | 13.0 | 55.7 | 3.0 | 13.1 |
| 43 | 3-Br-Phenyl | O | $CF_3$ | H | oil | 46.9 | 2.4 | 10.9 | 46.2 | 2.6 | 8.9 |
| 44 | 4-F-Phenyl | O | $CF_3$ | H | 63-65 | 55.7 | 2.8 | 13.0 | 55.6 | 2.8 | 12.9 |
| 45 | 2-F-Phenyl | O | $CF_3$ | H | | 55.7 | 2.8 | 13.0 | 55.7 | 2.9 | 13.1 |
| 46 | Phenyl | S | $CF_3$ | H | oil | 56.1 | 3.1 | 13.1 | 56.2 | 3.4 | 11.8 |

Example 47

Herbicidal Activity

To evaluate their herbicidal activity, compounds according to the invention were tested using as representative range of plants: maize, *Zea mays* (Mz); rice *Oryza sativa* (R); barnyard grass, *Echinochloa crusgalli* (BG); oat, *Avena sativa* (O); linseed, *Linum usitatissisum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0—9 scale. A rating 0 indicates growth as untreated control, a rating 9 indicates death. An increaase of 1 unit on the linear scale approximates to a 10% increase in the level of effect.

The results of the tests are set out in Table 3.

Table 3

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 3 | 3 | 3 | 3 | 0 | 4 | 5 | 3 | 4 | 5 | 5 | 4 | 5 | 6 | 8 | 8 | 6 | 5 | 1 | 0 | 4 | 2 | 2 | 3 | 3 | 0 |
| | | | | | | | | | 1 | 4 | 2 | 3 | 2 | 3 | 5 | 4 | 4 | 0 | 0 | 2 | 1 | 0 | 1 | 2 | 0 |
| 2 | 5 | 5 | 6 | 7 | 6 | 6 | 6 | 4 | 5 | 5 | 4 | 7 | 7 | 7 | 8 | 9 | 8 | 6 | 4 | 9 | 7 | 5 | 8 | 9 | 4 |
| | | | | | | | | | 1 | 3 | 2 | 5 | 5 | 6 | 8 | 8 | 7 | 4 | 2 | 9 | 5 | 1 | 5 | 8 | 0 |
| 1 | 0 | 0 | 0 | 0 | 3 | 3 | 0 | 0 | 5 | 2 | 0 | 3 | 2 | 4 | 5 | 2 | 3 | 0 | 0 | 0 | 0 | 0 | 3 | 0 | 0 |
| | | | | | | | | | 1 | 0 | 0 | 0 | 0 | 3 | 3 | 0 | 3 | 0 | 0 | 0 | 0 | 0 | 0 | 0 | 0 |
| 4 | 0 | 0 | 0 | 0 | 0 | 0 | 0 | 0 | 5 | 0 | 0 | 3 | 0 | 4 | 8 | 7 | 4 | 0 | 0 | 0 | 0 | 0 | 0 | 0 | 0 |
| | | | | | | | | | 1 | 0 | 0 | 1 | 0 | 2 | 3 | 2 | 3 | 0 | 0 | 0 | 0 | 0 | 0 | 0 | 0 |

Table 3 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 38 | 0 | 0 | 0 | 0 | 0 | 0 | 0 | 0 | 5 | 1 | 0 | 0 | 2 | 6 | 6 | 4 | 4 | 0 | 0 | 2 | 3 | 2 | 6 | 0 | 2 |
| | | | | | | | | | 1 | 0 | 0 | 0 | 0 | 2 | 3 | 2 | 2 | 0 | 0 | 0 | 0 | 0 | 1 | 0 | 0 |
| 39 | 5 | 5 | 6 | 6 | 3 | 3 | 3 | 4 | 5 | 7 | 4 | 8 | 6 | 7 | 7 | 8 | 6 | 6 | 6 | 9 | 7 | 8 | 8 | 9 | 3 |
| | | | | | | | | | 1 | 5 | 3 | 6 | 4 | 6 | 6 | 7 | 6 | 4 | 4 | 9 | 5 | 6 | 4 | 7 | 1 |
| 5 | 5 | 4 | 5 | 6 | 4 | 4 | 4 | 2 | 5 | 5 | 5 | 7 | 7 | 8 | 9 | 8 | 5 | 5 | 4 | 9 | 8 | 6 | 9 | 8 | 4 |
| | | | | | | | | | 1 | 3 | 2 | 4 | 5 | 7 | 8 | 5 | 3 | 3 | 2 | 9 | 5 | 5 | 5 | 2 | 1 |
| 8 | 2 | 2 | 5 | 6 | 3 | 2 | 2 | 0 | 5 | 5 | 5 | 7 | 7 | 8 | 9 | 7 | 4 | 3 | 4 | 8 | 6 | 4 | 7 | 6 | 1 |
| | | | | | | | | | 1 | 2 | 3 | 6 | 6 | 8 | 8 | 6 | 4 | 2 | 3 | 7 | 4 | 2 | 2 | 2 | 0 |

Table 3 continued....

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 9 | 1 | 0 | 3 | 4 | 0 | 1 | 1 | 0 | 5 | 4 | 4 | 7 | 7 | 8 | 8 | 8 | 4 | 0 | 3 | 9 | 5 | 2 | 3 | 3 | 1 |
| | | | | | | | | | 1 | 3 | 2 | 4 | 5 | 7 | 8 | 5 | 4 | 0 | 1 | 4 | 3 | 0 | 1 | 0 | 0 |
| 6 | 5 | 3 | 6 | 7 | 5 | 3 | 2 | 2 | 5 | 5 | 4 | 7 | 7 | 8 | 8 | 8 | 4 | 4 | 3 | 9 | 7 | 5 | 6 | 8 | 2 |
| | | | | | | | | | 1 | 2 | 1 | 4 | 5 | 7 | 8 | 5 | 4 | 1 | 1 | 7 | 2 | 2 | 3 | 1 | 0 |
| 7 | 0 | 0 | 0 | 0 | 0 | 0 | 0 | 0 | 5 | 3 | 2 | 4 | 4 | 5 | 5 | 6 | 5 | 3 | 1 | 8 | 6 | 4 | 5 | 5 | 2 |
| | | | | | | | | | 1 | 1 | 0 | 1 | 0 | 3 | 3 | 2 | 2 | 0 | 0 | 3 | 2 | 0 | 0 | 0 | 0 |
| 10 | 0 | 0 | 0 | 0 | 0 | 0 | 0 | 0 | 5 | 2 | 1 | 5 | 5 | 5 | 6 | 3 | 4 | 1 | 0 | 7 | 4 | 0 | 2 | 1 | 0 |
| | | | | | | | | | 1 | 1 | 0 | 1 | 1 | 2 | 4 | 1 | 2 | 0 | 0 | 2 | 1 | 0 | 0 | 0 | 0 |
| 12 | | | | | | | | | 5 | 7 | 4 | 7 | 6 | 7 | 5 | 5 | 6 | 5 | 4 | 8 | 5 | 3 | 4 | 6 | 4 |
| | | | | | | | | | 1 | 5 | 2 | 5 | 4 | 7 | 4 | 4 | 4 | 2 | 2 | 5 | 2 | | | | |

Table 3 continued....

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 13 | | | | | | | | | 5 | 4 | 3 | 5 | 3 | 4 | 3 | | 4 | | | | | | | | |
| | | | | | | | | | 1 | 2 | 3 | | | 2 | 2 | | 2 | | | | | | | | |
| 16 | | | | | | | | | 5 | 5 | 3 | 8 | 5 | 7 | 7 | 7 | 6 | 4 | 4 | 9 | 7 | 5 | 7 | 5 | |
| | | | | | | | | | 1 | 3 | 2 | 6 | 3 | 6 | 6 | 6 | 6 | 3 | 2 | 8 | 5 | 2 | 6 | 3 | |
| 17 | 5 | 4 | 5 | 5 | 3 | 3 | 4 | | 5 | 6 | | 6 | 3 | 7 | 7 | 4 | 7 | 4 | 3 | 7 | 6 | 6 | 8 | 5 | 2 |
| | | | | | | | | | 1 | 4 | | 4 | 2 | 6 | 7 | | 6 | 2 | 2 | 7 | 4 | 4 | 7 | 5 | |
| 19 | 1 | 2 | 4 | 3 | 8 | 3 | 2 | | 5 | 8 | 5 | 8 | 5 | 7 | 8 | 8 | 8 | 4 | 5 | 7 | 7 | 4 | 6 | 6 | 2 |
| | | | | | | | | | 1 | 5 | 4 | 7 | 3 | 5 | 6 | 7 | 7 | 2 | 4 | | 5 | 2 | 6 | 5 | 1 |

Table 3 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 20 | 6 | 5 | 6 | 4 | 6 | 6 | 6 | 3 | 5 | 4 | 1 | 5 | 4 | 7 | 6 | 7 | 7 | 2 | 0 | 4 | 4 | 5 | 4 | 7 | |
| | | | | | | | | | 1 | 2 | | 2 | 2 | 5 | 5 | 6 | 6 | | | 4 | 2 | 5 | 4 | 5 | |
| 21 | | | | | | | | | 5 | 6 | | 6 | 3 | 3 | 4 | 3 | 4 | | | | | | | | |
| | | | | | | | | | 1 | 4 | | 3 | 1 | 3 | 3 | 2 | 4 | | | | | | | | |
| 22 | | | | | | | | | 5 | 5 | 0 | 4 | | | 4 | 6 | 5 | | | | | | | | |
| | | | | | | | | | 1 | 2 | | 2 | | | 3 | 4 | 3 | | | | | | | | |
| 24 | 2 | 3 | 6 | 3 | 4 | 5 | 2 | 4 | 5 | 2 | 2 | 6 | 2 | 5 | 6 | 5 | 5 | 0 | 2 | 8 | 3 | 5 | 8 | 7 | 2 |
| | | | | | | | | | 1 | 2 | 2 | 6 | 1 | 4 | 5 | 4 | 5 | 0 | 0 | 3 | 0 | 2 | 6 | 3 | 0 |
| 23 | 4 | 6 | 6 | 5 | 6 | 5 | 4 | 3 | 5 | 4 | 2 | 7 | 3 | 6 | 6 | 6 | 5 | 3 | 3 | 8 | 4 | 4 | 8 | 8 | 4 |
| | | | | | | | | | 1 | 4 | 1 | 6 | 2 | 5 | 4 | 6 | 5 | 0 | 0 | 4 | 1 | 4 | 4 | 2 | 1 |

Table 3 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 25 | 3 | 2 | 2 | 4 | 4 | 6 | 5 | 4 | 5 | 3 | 0 | 2 | 2 | 4 | 5 | 5 | 6 | 0 | 0 | 0 | 3 | 3 | 6 | 4 | 2 |
| | | | | | | | | | 1 | 3 | 0 | 1 | 2 | 3 | 3 | 3 | 4 | 0 | 0 | 0 | 1 | 0 | 3 | 2 | 1 |
| 28 | | | | | | | | | 5 | 5 | 4 | 6 | 5 | 6 | 6 | 7 | 6 | 4 | 4 | 8 | 6 | 5 | 6 | 3 | 0 |
| | | | | | | | | | 1 | 3 | 2 | 4 | 3 | 5 | 5 | 5 | 4 | 3 | 2 | 7 | 2 | 0 | 5 | 0 | 0 |
| 29 | | | | | | | | | 5 | 4 | 3 | 4 | 4 | 6 | 4 | 0 | 3 | 2 | 0 | 5 | 0 | 0 | 2 | 0 | 0 |
| | | | | | | | | | 1 | 2 | 0 | 0 | 2 | 4 | 2 | 0 | 1 | 1 | 0 | 1 | 0 | 0 | 1 | 0 | 0 |
| 32 | | | | | | | | | 5 | 4 | 2 | 4 | 5 | 5 | 5 | 6 | 6 | 0 | 0 | 0 | 0 | 0 | 0 | 0 | 0 |
| | | | | | | | | | 1 | 2 | 1 | 2 | 2 | 1 | 3 | 4 | 3 | 0 | 0 | 0 | 0 | 0 | 0 | 0 | 0 |
| 33 | 0 | 0 | 0 | 0 | 0 | 0 | 0 | 0 | 5 | 4 | 0 | 4 | 2 | 0 | 4 | 5 | 1 | 0 | 0 | 0 | 0 | 0 | 0 | 0 | 0 |
| | | | | | | | | | 1 | 2 | 0 | 2 | 1 | 0 | 2 | 3 | 1 | 0 | 0 | 0 | 0 | 0 | 0 | 0 | 0 |

Table 3 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 34 | 0 | 0 | 0 | 0 | 0 | 0 | 0 | 0 | 5 | | | | | | | | | | | | | | | | |
| | | | | | | | | | 1 | 2 | 3 | 3 | 3 | 3 | 0 | 4 | 4 | 0 | 0 | 0 | 0 | 0 | 0 | 0 | 0 |
| 36 | 0 | 0 | 0 | 0 | 0 | 0 | 0 | 0 | 5 | 4 | 0 | 4 | 3 | 5 | 8 | 6 | 5 | 0 | 0 | 0 | 0 | 0 | 0 | 0 | 0 |
| | | | | | | | | | 1 | 1 | 0 | 3 | 2 | 3 | 6 | 4 | 3 | 0 | 0 | 0 | 0 | 0 | 0 | 0 | 0 |
| 37 | 0 | 0 | 0 | 0 | 0 | 0 | 0 | 0 | 5 | 3 | 0 | 4 | 3 | 4 | 6 | 6 | 6 | 0 | 0 | 0 | 0 | 0 | 0 | 0 | 0 |
| | | | | | | | | | 1 | 2 | 0 | 2 | 1 | 2 | 4 | 5 | 4 | 0 | 0 | 0 | 0 | 0 | 0 | 0 | 0 |
| 42 | 4 | 3 | 4 | 3 | 5 | 3 | 3 | 4 | 5 | 6 | 3 | 8 | 6 | 7 | 7 | 9 | 8 | 4 | 3 | 9 | 7 | 7 | 8 | 9 | 3 |
| | | | | | | | | | 1 | 3 | 1 | 5 | 3 | 6 | 6 | 8 | 8 | 1 | 0 | 7 | 5 | 3 | 6 | 5 | 0 |
| 43 | 0 | 0 | 1 | 1 | 2 | 1 | 1 | 1 | 5 | 4 | 0 | 5 | 5 | 5 | 8 | 6 | 5 | 0 | 0 | 6 | 4 | 3 | 7 | 6 | 0 |
| | | | | | | | | | 1 | 2 | 0 | 2 | 3 | 4 | 7 | 5 | 4 | 0 | 0 | 2 | 0 | 0 | 3 | 6 | 0 |

Table 3 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 44 | 0 | 0 | 3 | 2 | 2 | 1 | 2 | 0 | 5 | 2 | 1 | 3 | 4 | 5 | 8 | 6 | 5 | 1 | 0 | 4 | 2 | 3 | 6 | 5 | 0 |
| | | | | | | | | | 1 | 0 | 0 | 2 | 2 | 4 | 6 | 6 | 4 | 0 | 0 | 0 | 0 | 0 | 0 | 0 | 0 |
| 45 | 0 | 0 | 0 | 0 | 0 | 0 | 4 | 2 | 5 | 4 | 4 | 5 | 4 | 6 | 6 | 6 | 5 | 0 | 0 | 0 | 0 | 0 | 0 | 0 | 0 |
| | | | | | | | | | 1 | 3 | 2 | 3 | 2 | 5 | 5 | 5 | 3 | 0 | 0 | 0 | 0 | 0 | 0 | 0 | 0 |

**Claims for the Contracting States: BE CH DE FR GB IT LI LU NL SE**

1. A herbicidal composition which comprises a carrier and, as active ingredient, a N-aryl triazole of the general formula I:—

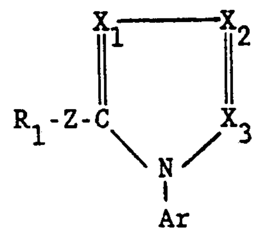

I

wherein $X_3$ and one of $X_1$ and $X_2$ represent a nitrogen atom and the other of $X_1$ and $X_2$ represents a group $C—R_2$;

Z represents an imino group, or an oxygen or sulphur atom;

$R_1$ represents an optionally substituted alkyl, aralkyl, alkenyl, cycloalkyl, cycloalkenyl, heteroaryl or aryl group;

$R_2$ represents a hydrogen or halogen atom or an optionally substituted aryl or alkyl group;

and Ar represents an optionally substituted aryl group.

2. Composition as claimed in claim 1, wherein Z represents an oxygen atom.

3. Composition as claimed in claim 1 or 2, wherein $R_1$ represents an alkyl or haloalkyl group of up to 4 carbon atoms, a cycloalkyl or cycloalkenyl group of 5 to 8 carbon atoms, a pyridyl group, a naphthyl group, or a phenyl group optionally substituted by a fluorine, chlorine or bromine atom or a hydroxy, cyano, methyl, trifluoromethyl, methoxy or methoxycarbonyl group; and $R_2$ represents a hydrogen or halogen atom, an alkyl group of 1 to 4 carbon atoms, or a phenyl group.

4. Composition as claimed in claim 1, 2 or 3, wherein Ar represents a phenyl group optionally bearing a trifluoromethyl group at the 3 position.

5. A N-aryl triazole of the formula I as shown in Claim 1, wherein the substituents have the meanings defined in claim 1, subject to the proviso that when $X_2$ represents CPhenyl and $R_1Z$ represents phenoxy, then Ar is not unsubstituted phenyl; and when $X_2$ represents CH, then Ar is not a 2,4-dichloro-5-methoxy phenyl group.

6. Process for the preparation of a compound as defined in claim 5, in which Z is an oxygen or sulphur atom, which comprises reacting a halo-triazole of formula II:

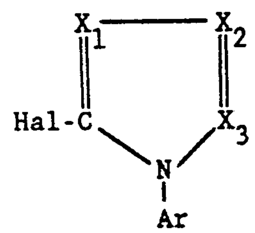

II

with an alcohol or mercaptan of formula $R_1ZH$ or an alkali metal salt of such alcohol, wherein $R_1$, Ar, $X_1$, $X_2$, $X_3$ and Z are as defined in claim 5 and Hal denotes a halogen atom.

7. Process as claimed in claim 6, wherein the halo-triazole is a chloro-triazole of formula II in which Hal denotes a chlorine atom, and the reaction is carried out in an organic solvent and in the presence of a base.

8. Process for the preparation of a compound as defined in claim 1, wherein $X_1$ represents the group $CR_2$ and Z is oxygen, which comprises reacting an aromatic azide of formula $ArN_3$ with an acetylenic derivative of formula $R_1OC{\equiv}CH$ or alternatively successively with a dialkyl malonate, phosphorus pentachloride, a phenol of formula $R_1OH$, and thereafter hydrolising and heating to effect decarboxylation.

9. Method of combating undesired plant growth at a locus, which comprises treating the locus with a composition as claimed in any one of claims 1 to 4 or a compound as claimed in claim 5.

10. Use as a herbicide of a N-aryl triazole as defined in any one of claims 1 to 5.

**Claims for the Contracting State: AT**

1. A herbicidal composition which comprises a carrier and, as active ingredient, a N-aryl triazole of the general formula I:—

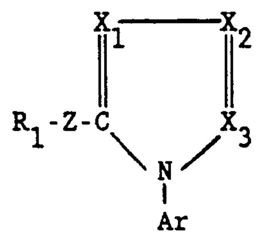

I

wherein $X_3$ and one of $X_1$ and $X_2$ represent a nitrogen atom and the other of $X_1$ and $X_2$ represents a group $C—R_2$;

Z represents an imino group, or an oxygen or sulphur atom;

$R_1$ represents an optionally substituted alkyl, aralkyl, alkenyl, cycloalkyl, cycloalkenyl, heteroaryl or aryl group;

$R_2$ represents a hydrogen or halogen atom or an optionally substituted aryl or alkyl group;

and Ar represents an optionally substituted aryl group.

2. Composition as claimed in claim 1, wherein Z represents an oxygen atom.

3. Composition as claimed in claim 1 or 2, wherein $R_1$ represents an alkyl or haloalkyl group of up to 4 carbon atoms, a cycloalkyl or cycloalkenyl group of 5 to 8 carbon atoms, a pyridyl group, a naphthyl group, or a phenyl group optionally substituted by a fluorine, chlorine or bromine atom or a hydroxy, cyano, methyl, trifluoromethyl, methoxy or methoxycarbonyl group; and $R_2$ represents a hydrogen or halogen atom, an alkyl group of 1 to 4 carbon atoms, or a phenyl group.

4. Composition as claimed in claim 1, 2 or 3, wherein Ar represents a phenyl group optionally bearing a trifluoromethyl group at the 3 position.

5. Process for the preparation of a compound as defined in claim 1 in which Z is an oxygen or sulphur atom, which comprises reacting a halo-triazole of formula II:

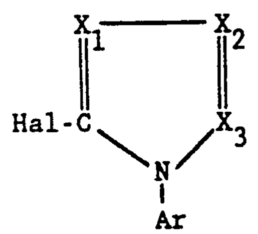

II

with an alcohol or mercaptan of formula $R_1ZH$ or an alkali metal salt of such alcohol, wherein $R_1$, Ar, $X_1$, $X_2$, $X_3$ and Z are as defined in claim 1 and Hal denotes a halogen atom.

6. Process as claimed in claim 5, wherein the halo-triazole is a chloro-triazole of formula II in which Hal denotes a chlorine atom, and the reaction is carried out in an organic solvent and in the presence of a base.

7. Process for the preparation of a compound as defined in claim 1, wherein $X_1$ represents the group $CR_2$ and Z is oxygen, which comprises reacting an aromatic azide of formula $ArN_3$ with an acetylenic derivative of formula $R_1OC{\equiv}CH$ or alternatively successively with a dialkyl malonate, phosphorus pentachloride, a phenol of formula $R_1OH$, and thereafter hydrolising and heating to effect decarboxylation.

8. Method of combating undesired plant growth at a locus, which comprises treating the locus with a composition as claimed in any one of claims 1 to 4 or a N-aryltriazole as defined in any one of claims 1 to 4.

9. Use as a herbicide of a N-aryl triazole as defined in any one of claims 1 to 4.

**Patentansprüche für die Vertragsstaaten: BE CH DE FR GB IT LI LU NL SE**

1. Herbizide Zusammensetzung, welche einen Träger und als wirksamen Bestandteil ein N-Aryltriazol der allgemeinen Formel I:

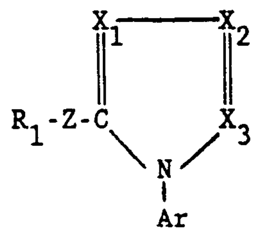

I

umfaßt, worin

$X_3$ und einer der Reste von $X_1$ und $X_2$ ein Stickstoffatom bedeuten und der andere Rest von $X_1$ und $X_2$ eine Gruppe $C—R_2$ darstellt;

Z eine Iminogruppe oder ein Sauerstoff- oder Schwefelatom darstellt;

$R_1$ eine gegebenenfalls substituierte Alkyl-, Aralkyl-, Alkenyl-, Cycloalkyl-, Cycloalkenyl-, Heteroaryl- oder Arylgruppe darstellt;

$R_1$ ein Wasserstoff- oder Halogenatom oder eine gegebenenfalls substituierte Aryl- oder Alkylgruppe bedeutet; und

Ar eine gegebenenfalls substituierte Arylgruppe darstellt,

2. Zusammensetzung nach Anspruch 1, worin Z ein Sauerstoffatom bedeutet.

3. Zusammensetzung nach Anspruch 1 oder 2, worin

$R_1$ eine Alkyl- oder Halogenalkylgruppe mit bis zu 4 Kohlenstoffatomen, eine Cycloalkyl- oder Cycloalkenylgruppe mit 5 bis 8 Kohlenstoffatomen, eine Pyridylgruppe, eine Naphthylgruppe oder eine Phenylgruppe bedeutet, die gegebenenfalls durch ein Fluor-, Chlor- oder Bromatom oder eine Hydroxy-, Cyano-, Methyl-, Trifluormethyl-, Methoxy- oder Methoxycarbonylgruppe substituiert ist; und

$R_2$ ein Wasserstoff- oder Halogenatom, eine Alkylgruppe mit 1 bis 4 Kohlenstoffatomen oder eine Phenylgruppe bedeutet.

4. Zusammensetzung nach Anspruch 1, 2 oder 3, worin Ar eine Phenylgruppe darstellt, die gegebenenfalls an der 3-Stellung eine Trifluormethylgruppe aufweist.

5. Ein N-Aryltriazol der Formel I, wie in Anspruch 1 dargestellt, worin die Substituenten die in Anspruch 1 definierten Bedeutungen mit den Maßgaben besitzen, daß dann, wenn $X_2$ CPhenyl bedeutet und $R_1Z$ Phenoxy darstellt, Ar nicht unsubstituiertes Phenyl ist; und daß dann, wenn $X_2$ für CH steht, Ar night eine 2,4-Dichlor-5-methoxyphenylgruppe ist.

6. Verfahren zur Herstellung einer Verbindung, wie in Anspruch 5 definiert, worin Z ein Sauerstoff- oder Schwefelatom bedeutet, welches ein Umsetzen eines Halogentriazols der Formel II:

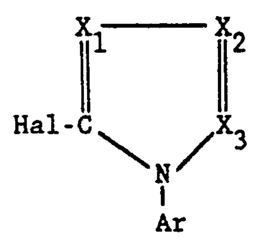

II

mit einem Alkohol oder einem Mercaptan der Formel $R_1ZH$ oder einem Alkalimetallsalz eines solchen Alkohols umfaßt, worin $R_1$, Ar, $X_1$, $X_2$, $X_3$ und Z wie in Anspruch 5 definiert sind und Hal ein Halogenatom bezeichnet.

7. Verfahren nach Anspruch 6, worin das Halogentriazol ein Chlortriazol der Formel II ist, worin Hal ein Chloratom bezeichnet, und die Reaktion in einem organischen Lösungsmittel in Anwesenheit einer Base ausgeführt wird.

8. Verfahren zur Herstellung einer Verbindung, wie in Anspruch 1 definiert, worin $X_1$ die Gruppe $CR_2$ bedeutet und Z für Sauerstoff steht, welches ein Umsetzen eines aromatischen Azids der Formel $ArN_3$ mit einem Acetylenderivat der Formel $R_1OC{\equiv}CH$ oder in alternativer Weise aufeinanderfolgend mit einem Dialkylmalonat, Phosphorpentachlorid, einem Phenol der Formel $R_1OH$ und anschließend Hydrolysieren und Erwärmen zur Bewirkung einer Decarboxylierung umfaßt.

9. Verfahren zur Bekämpfung von unerwünschten Pflanzenwuchs an einem Ort, welches ein Behandeln des Ortes mit einer Zusammensetzung, wie in einem der Ansprüche 1 bis 4 beansprucht, oder mit einer Verbindung, wie in Anspruch 5 beansprucht, umfaßt.

10. Verwendung eines N-Aryltriazols, wie in einem der Ansprüche 1 bis 5 definiert, als ein Herbizid.

**Patentansprüche für den Vertragsstaat: AT**

1. Herbizide Zusammensetzung, welche einen Träger und als wirksamen Bestandteil ein N-Aryltriazol der allgemeinen Formel I:

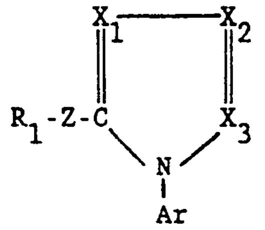

I

umfaßt, worin

$X_3$ und einer der Reste von $X_1$ und $X_2$ ein Stickstoffatom bedeuten und der andere Rest von $X_1$ und $X_2$ eine Gruppe $C—R_2$ darstellt;

Z eine Iminogruppe oder ein Sauerstoff- oder Schwefelatom darstellt;

$R_1$ eine gegebenenfalls substituierte Alkyl-, Aralkyl-, Alkenyl-, Cycloalkyl-, Cycloalkenyl-, Heteroaryl- oder Arylgruppe darstellt;

$R_1$ ein Wasserstoff- oder Halogenatom oder eine gegebenenfalls substituierte Aryl- oder Alkylgruppe bedeutet; und

Ar eine gegebenenfalls substituierte Arylgruppe darstellt,

2. Zusammensetzung nach Anspruch 1, worin Z ein Sauerstoffatom bedeutet.

3. Zusammensetzung nach Anspruch 1 oder 2, worin

$R_1$ eine Alkyl- oder Halogenalkylgruppe mit bis zu 4 Kohlenstoffatomen, eine Cycloalkyl- oder Cycloalkenylgruppe mit 5 bis 8 Kohlenstoffatomen, eine Pyridylgruppe, eine Naphthylgruppe oder eine Phenylgruppe bedeutet, die gegebenenfalls durch ein Fluor-, Chlor- oder Bromatom oder eine Hydroxy-, Cyano-, Methyl-, Trifluormethyl-, Methoxy- oder Methoxycarbonylgruppe substituiert ist; und

$R_2$ ein Wasserstoff- oder Halogenatom, eine Alkylgruppe mit 1 bis 4 Kohlenstoffatomen oder eine Phenylgruppe bedeutet.

4. Zusammensetzung nach Anspruch 1, 2 oder 3, worin Ar eine Phenylgruppe darstellt, die gegebenenfalls an der 3-Stellung eine Trifluormethylgruppe aufweist.

5. Verfahren zur Herstellung einer Verbindung, wie in Anspruch 1 definiert, worin Z ein Sauerstoff- oder Schwefelatom bedeutet, welches ein Umsetzen eines Halogentriazols der Formel II:

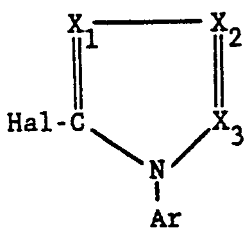

II

mit einem Alkohol oder Mercaptan der Formel $R_1ZH$ oder einem Alkalimetallsalz eines solchen Alkohols umfaßt, worin $R_1$, Ar, $X_1$, $X_2$, $X_3$ und Z wie in Anspruch 1 definiert sind und Hal ein Halogenatom bezeichnet.

6. Verfahren nach Anspruch 5, worin das Halogentriazol ein Chlortriazol der Formel II ist, worin Hal ein Chloratom bezeichnet, und die Reaktion in einem organischen Lösungsmittel und in Anwesenheit einer Base ausgeführt wird.

7. Verfahren zur Herstellung einer Verbindung, wie in Anspruch 1 definiert, worin $X_1$ die Gruppe $CR_2$ darstellt und Z Sauerstoff bedeutet, welches ein Umsetzen eines aromatischen Azids der Formel $ArN_3$ mit einem Acetylenderivat der Formel $R_1OC{\equiv}CH$ oder in alternativer Weise aufeinanderfolgend mit einem Dialkylmalonat, Phosphorpentachlorid, einem Phenol der Formel $R_1OH$, und anschließend ein Hydrolysieren und Erwärmen zur Bewirkung einer Decarboxylierung umfaßt.

8. Verfahren zur Bekämpfung von unerwünschten Pflanzenwuchs an einem Ort, welches ein Behandeln des Ortes mit einer Zusammensetzung, wie in einem der Ansprüche 1 bis 4 beansprucht, oder einem N-Aryltriazol, wie in einem der Ansprüche 1 bis 4 definiert, umfaßt.

9. Verwendung eines N-Aryltriazols, wie in einem der Ansprüche 1 bis 4 definiert, als ein Herbizid.

**Revendications pour les Etats contractants: BE CH DE FR GB IT LU NL SE**

1. Une composition herbicide qui comprend un véhicule et, comme ingrédient actif, un N-aryl triazole de la formule générale I:

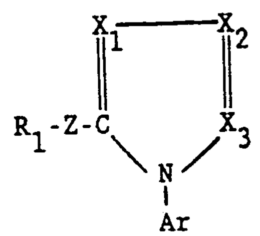

I

où $X_3$ et un de $X_1$ et $X_2$ représentent chacun un atome d'azote ou l'autre de $X_1$ et $X_2$ représente un groupe $C—R_2$;

Z représente un groupe imino ou un atome d'oxygène ou de soufre;

$R_1$ représente un groupe alcoyle, aralcoyle, alcényle, cycloalcoyle, cycloalcényle, hétéroaryle ou aryle éventuellement substitué;

$R_2$ représente un atome d'hydrogène ou d'un halogène ou un groupe aryle ou alcoyle éventuellement substitué;

et Ar représente un groupe aryle éventuellement substitué.

2. Composition selon la revendication 1, dans laquelle Z représente un atome d'oxygène.

3. Composition selon la revendication 1 ou 2, dans laquelle $R_1$ représente un groupe alcoyle ou haloalcoyle ayant jusqu'à 4 atomes de carbone, un groupe cycloalcoyle ou cycloalcényle de 5 à 8 atomes de carbone, un groupe pyridyle, un groupe naphtyle ou un groupe phényle éventuellement substitué par un atome de fluor, de chlore ou de brome ou par un groupe hydroxy, cyano, méthyle, trifluorométhyle, méthoxy ou méthoxycarbonyle; et $R_2$ représente un atome d'hydrogène ou d'un halogène, un groupe alcoyle de 1 à 4 atomes de carbone ou un groupe phényle.

4. Composition selon la revendication 1, 2 ou 3, dans laquelle Ar représente un groupe phényle portant éventuellement un groupe trifluorométhyle à la position 3.

5. Un N-aryl triazole de la formule I telle que représentée dans la revendication 1, où les substituants ont les significations définies dans la revendication 1, avec les conditions que quand $X_2$ représente un groupe C-phényle et $R_1Z$ représente un groupe phénoxy, alors Ar ne doit pas être un groupe phényle substitué; et quand $X_2$ représente CH, alors Ar ne doit pas être un groupe 2,4-dichloro-5-méthoxy phényle.

6. Procédé pour la préparation d'un composé tel que défini dans la revendication 5, dans lequel Z est un atome d'oxygène ou de soufre, qui comprend la réaction d'un halo-triazole de formule II:

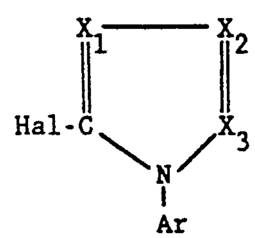

II

avec un alcool ou mercaptan de formule $R_1ZH$ ou avec un sel de métal alcalin d'un tel alcool, où $R_1$, Ar, $X_1$, $X_2$, $X_3$ et Z sont tels que définis dans la revendication 5 et Hal représente un atome d'halogène.

7. Procédé selon la revendication 6, dans lequel l'halo-triazole est un chloro-triazole de formule II où Hal représente un atome de chlore, et la réaction est conduite dans un solvant organique et en présence d'une base.

8. Procédé pour la préparation d'un composé tel que défini dans la revendication 1, dans lequel $X_1$ représente le groupe $CR_2$ et Z est de l'oxygène, qui comprend la réaction d'un azide aromatique de formule $ArN_3$ avec un dérivé acétylénique de formule $R_1OC{\equiv}CH$ ou en variante successivement avec un malonate de dialcoyle, du pentachlorure de phosphore, un phénol de formule $R_1OH$, avec ensuite hydrolyse et chauffage pour effectuer une décarboxylation.

9. Méthode de lutte contre la croissance de plantes indésirables en un lieu, qui comprend le traitement du lieu avec une composition selon l'une quelconque des revendications 1 à 4 ou avec un composé selon la revendication 5.

10. Utilisation comme herbicide d'un N-aryl triazole tel que défini dans l'une quelconque des revendications 1 à 5.

**Revendications pour l'Etat contractant: AT**

1. Une composition herbicide qui comprend un véhicule et, comme ingrédient actif, un N-aryl triazole de la formule générale I:

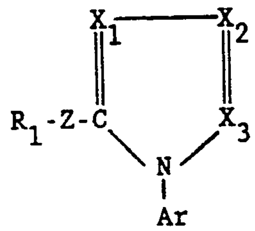

I

où $X_3$ et un de $X_1$ et $X_2$ représentent chacun un atome d'azote et l'autre de $X_1$ et $X_2$ représente un groupe $C—R_2$;

Z représente un groupe imino ou un atome d'oxygène ou de soufre;

$R_1$ représente un groupe alcoyle, aralcoyle, alcényle, cycloalcoyle, cycloalcényle, hétéroaryle ou aryle éventuellement substitué;

$R_2$ représente un atome d'hydrogène ou d'un halogène ou un groupe aryle ou alcoyle éventuellement substitué;

et Ar représente un groupe aryle éventuellement substitué.

2. Composition selon la revendication 1, dans laquelle Z représente un atome d'oxygène.

3. Composition selon la revendication 1 ou 2, dans laquelle $R_1$ représente un groupe alcoyle ou haloalcoyle ayant jusqu'à 4 atomes de carbone, un groupe cycloalcoyle ou cycloalcényle de 5 à 8 atomes de carbone, un groupe pyridyle, un groupe naphtyle ou un groupe phényle éventuellement substitué par un atome de fluor, de chlore ou de brome ou par un groupe hydroxy, cyano, méthyle, trifluorométhyle, méthoxy ou méthoxycarbonyle; et $R_2$ représente un atome d'hydrogène ou d'un halogène, un groupe alcoyle de 1 à 4 atomes de carbone ou un groupe phényle.

4. Composition selon la revendication 1, 2 ou 3, dans laquelle Ar représente un groupe phényle portant éventuellement un groupe trifluorométhyle à la position 3.

5. Procédé pour la préparation d'un composé tel que défini dans la revendication 1 dans lequel Z est un atome d'oxygène ou de soufre, qui comprend la réaction d'un halo-triazole de formule II:

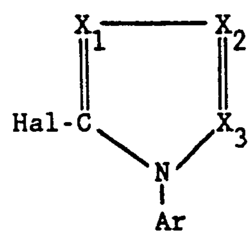

II

avec un alcool ou mercaptan de formule $R_1ZH$ ou avec un sel de métal alcalin d'un tel alcool, où $R_1$, Ar, $X_1$, $X_2$, $X_3$ et Z sont tels que définis dans la revendication 1 et Hal représente un atome d'halogène.

6. Procédé selon la revendication 5, dans lequel l'halo-triazole est un chloro-triazole de formule II où Hal représente un atome de chlore et la réaction est conduite dans un solvant organique et en présence d'une base.

7. Procédé pour la préparation d'un composé tel que défini dans la revendication 1, dans lequel $X_1$ représente le groupe $CR_2$ et Z est de l'oxygène, qui comprend la réaction d'un azide aromatique de formule $ArN_3$ avec un dérivé acétylénique de formule $R_1OC{\equiv}CH$ ou en variante successivement avec un malonate de dialcoyle, du pentachlorure de phosphore, un phénol de formule $R_1OH$, avec ensuite hydrolyse et chauffage pour effectuer une décarboxylation.

8. Méthode de lutte contre la croissance de plantes indésirables en un lieu, qui comprend le traitement du lieu avec une composition selon l'une quelconque des revendicaations 1 à 4 ou avec un N-aryltriazole tel que défini dans l'une quelconque des revendications 1 à 4.

9. Utilisation comme herbicide d'un N-aryl triazole tel que défini dans l'une quelconque des revendications 1 à 4.